# EUROPEAN PATENT SPECIFICATION

(11) **EP 1 430 109 B1**
(45) Date of publication and mention of the grant of the patent: **04.01.2012**
(21) Application number: 02782795.5
(22) Date of filing: 25.09.2002
(51) Int. Cl.: C12N 1/20

(54) **CULTIVATION OF MYCOBACTERIA**
SUSPENSIONSKULTUR VON MYKOBAKTERIEN
CULTURE DE MYCOBACTERIES

(30) Priority: 27.09.2001 EP 01123146
(43) Date of publication of application: 23.06.2004
(73) Proprietor: Novartis Vaccines and Diagnostics GmbH, 35041 Marburg (DE)
(72) Inventor: DIETRICH, Guido, 1970 Wezembeek-Oppem (BE); HUNDT, Erika, 35041 Marburg (DE); WEBER, Heinz, 35085 Ebsdorfergrund-Dreihausen (DE)
(74) Representative: Vossius & Partner
(86) International application number: PCT/EP2002/010772
(87) International publication number: WO 2003/029478

(56) References cited:
- US-A- 4 358 538
- PATENT ABSTRACTS OF JAPAN vol. 004, no. 113 (C-021), 13 August 1980 (1980-08-13) -& JP 55 071489 A (MITSUI TOATSU CHEM INC), 29 May 1980 (1980-05-29)
- JAMES B W ET AL: "THE PHYSIOLOGY AND PATHOGENICITY OF MYCOBACTERIUM TUBERCULOSIS GROWN UNDER CONTROLLED CONDITIONS IN A DEFINED MEDIUM" JOURNAL OF APPLIED MICROBIOLOGY, OXFORD, GB, vol. 88, no. 4, 2000, pages 669-671, XP000929200 ISSN: 1364-5072
- GHEORGHIU M ET AL: "The effects of dispersed or surface grown cultures, manufacture and control methods on BCG standardization." DEVELOPMENTS IN BIOLOGICAL STANDARDIZATION. SWITZERLAND 1986, vol. 58 ( Pt A), 1986, pages 191-205, XP001155816 ISSN: 0301-5149
- NYABENDA J ET AL: "THE PRODUCTION OF MYCOBACTERIAL ANTIGENS BY HOMOGENEOUS CULTURE IN A FERMENTOR" JOURNAL OF BIOLOGICAL STANDARDIZATION, vol. 16, no. 4, 1988, pages 259-268, XP009019720 ISSN: 0092-1157
- VARDAR-SUKAN F: "Foaming: consequences, prevention and destruction" BIOTECHNOLOGY ADVANCES, ELSEVIER PUBLISHING, BARKING, GB, vol. 16, no. 5-6, 11 September 1998 (1998-09-11), pages 913-948, XP004138782 ISSN: 0734-9750
- BLENKHARN J I ET AL: "A LOW-COST ANTIFOAM ADDITIVE FOR AGAR-BASED CULTURE MEDIA" JOURNAL OF APPLIED BACTERIOLOGY, vol. 63, no. 5, 1987, pages 465-468, XP009019919 ISSN: 0021-8847
- DIETRICH GUIDO ET AL: "Cultivation of Mycobacterium bovis BCG in bioreactors" JOURNAL OF BIOTECHNOLOGY, vol. 96, no. 3, 3 July 2002 (2002-07-03), pages 259-270, XP002259429 ISSN: 0168-1656

## Description

The present invention relates to a culture medium for dispersed mycobacteria comprising at least one detergent and an antifoam agent, whereby said antifoam agent is a member of the silicone family. Furthermore, methods for the cultivation of dispersed mycobacteria are disclosed which comprise growing said mycobacteria in the culture medium of the invention. In addition, the invention relates to a method for obtaining standardized mycobacterial cultures or purified protein derivates (PPD) which comprise the cultivation of mycobacteria in the culture medium disclosed herein.

Several documents are cited throughout the text of this specification. Each of the documents cited herein (including any manufacturer's specifications, instructions, etc.) are hereby incorporated by reference.

As early as 1927, Calmette and Guérin developed a live vaccine against tuberculosis caused by the intracellular bacterial pathogen *Mycobacterium tuberculosis* (Calmette and Guérin, 1909; Calmette, 1927). This so-called bacille Calmette-Guérin (BCG) vaccine is an attenuated strain of *Mycobacterium bovis* that infects both, human beings and cattle. BCG represents the world's most widely used live vaccine with more than two billion vaccinations (Bloom and Fine, 1994). The vaccine is cheap to produce, gives rise to a long-lived state of resistance and causes a limited incidence of notable side-effects (Bloom and Murray, 1992; Bloom and Fine, 1994). Worldwide, a single dose of BCG in newborn children significantly protects against the development of severe forms of childhood tuberculosis (Rodrigues et al., 1993). However, protection by BCG against pulmonary tuberculosis in adults, the most prevalent form of the disease, is highly variable (Clemens et al., 1983; Bloom and Fine, 1994; Fine, 1995; Kaufmann, 2000). As evaluated in large numbers of clinical trials, and in various populations and geographic regions, the calculated protective efficacy of BCG varies between 0 and 80 % (Rodrigues and Smith, 1990; WHO, 1979; Colditz et al., 1994; Roche et al., 1995). This variability could be due to variations and insufficient standardization in vaccine production (Milstien and Gibson, 1990; Bloom and Fine, 1994; Comstock, 1994; Fine, 1995).
Problems impairing the standardization of the BCG vaccine have been described in detail by Levy et al. (1968). The conventional BCG vaccine is produced as a surface pellicle in culture flasks. In general, the BCG bacteria are grown in 500 or 1000 ml flasks on the surface of liquid Sauton medium which contains inorganic salts, citric acid, asparagin and gycerol. These cultures require long time periods and allow only very limited opportunities for recording and correcting culture parameters. Therefore, reproducibility of cultures can not be guaranteed. Furthermore, the growth of BCG as surface veils leads to the formation of bacterial aggregates of variable size. Due to the high lipid content in their cell walls, mycobacteria form aggregates in culture rendering quality control testing of the BCG vaccine difficult. According to P.A. van Hemert, "...determination of "viable units" (by colony counting on *Löwenstein* medium) appeared to be impossible, because suspensions of B.C.G. always consist of clumps, with the clump size varying in an undefined and uncontrollable way..." (van Hemert, 1971). These problems make mechanical disruption of the bacterial aggregates necessary, e.g. by steel ball or glass ball milling. However, with this procedure it is difficult to achieve a single cell suspension of live bacilli, therefore the determination of numbers of colony forming units (CFU) does not always represent actual numbers of live bacilli due to nondisrupted clumps. Moreover, the bead-milling process causes death or physical stress of a variable number of bacilli and hamperes the exact quantification and quality control of the final product due to an undefined proportion of harvested bacteria which are nonviable under these production conditions (Levy et al., 1968; Milstien and Gibson, 1990). The proportion of living and dead bacilli is an important determinant of BCG vaccine quality, influences the immunogenicity of the BCG vaccine and could also account for undesired side effects like variable suppurative adenitis occurring in vaccinated newborns (Milstien and Gibson, 1990; Gheorghiu et al., 1988). The classical production process instead gives rise to a vaccine preparation containing as few as 30% live BCG bacilli. Formation of aggregates and varying proportions of living bacilli make the quality control - especially the determination of numbers of live bacilli - very difficult. Bacterial aggregates and single bacilli exhibit a similar capacity to form colonies, leading to high standard deviations when determining the numbers of CFU (Gheorghiu et al., 1988). Enumeration of culturable BCG particles is subject to considerable variability even when standard procedures are applied rigorously (Levy et al., 1968; Milstien and Gibson, 1990). Experiments based on the measurements of bioluminescence (proportional to the adenosine triphosphate content) are reliable markers for viability, but require an exact determination of the adenosine triphosphate content per culturable particle (Milstien and Gibson, 1990). As an alternative to the growth of *M*. *bovis* BCG as surface veil, submersed growth in variable culture vessels was proposed. The preparation of dispersed-grown *M*. *bovis* BCG was first described by Dubos and Fenner (Dubos and Fenner, 1950; Fenner and Dubos, 1950) and later by several others (Kim, 1977; Sirks et al., 1971; Ungar et al., 1962; Gheorghiu et al., 1986 and 1988). These cultures yield some viable single cells, but nevertheless still resulted in clumps which had to be dispersed sonically (Gheorghiu, 1988). However, this method of bacterial dispersion leads to the death of a high proportion of bacterial cells (Gheorghiu, 1988). Homogenous culture in Wheaton double-side-arm bottles was developed for production of BCG for use in cancer immunotherapy (Kim, 1977). Gheorgiu et al. (1988) found that dispersed-grown BCG vaccine contains a higher proportion of single bacilli and only few aggregates. The dispersed grown bacteria also expressed higher viability and heat stability as compared to the classical surface-grown bacteria (Gheorgiu et al., 1988), yet the ratio of viable to dead organisms was low which further decreased after freeze-drying, to as little as 5 %. However, the immunogenicity and protective efficacy of the dispersed-grown vaccine was superior to the classical vaccine preparation. Finally, homogenous cultures of *M*. *bovis* BCG were performed in small-scale bioreactors (van Hemert, 1971; Nyabenda et al., 1988). Van Hemert cultured *M*. *bovis* BCG in Ungar medium containing Tween 80 or Triton WR 1339 as dispersing agents. While with Triton WR 1339, the average size of bacterial aggregates was estimated to be about 100 bacilli per clump, this was reduced to only 10 bacilli per clump when Tween 80 was used. Under these conditions, standardization of the process was merely achieved to some extent, leading to a reduced variation of the numbers of viable cells (van Hemert, 1971). However, the protocol provided by van Hemert still leads to an uncontrollable variation in the clump size (up to a factor of 10) making viable counting almost impossible (van Hemert, 1971). Hence, the method provided by van Hemert only allows determination of the bacillary mass, but not the numbers of live bacilli. Furthermore, the method provided by van Hemert does not allow stirring at a rate above 200 rpm; for example, stirring at 400 rpm was detrimental to the bacteria. Accordingly, good mixing of the culture in the bioreactor cannot be achieved by this method. The cultivation of *M*. *bovis* BCG in bioreactors has been applied to the production of Purified Protein Derivative (PPD) (Nyabenda et al., 1988). Cultures were successfully grown in Sauton medium, bacterial growth curves, pH changes in the medium and protein content of culture filtrates were comparable to static cultures as surface veils. The PPD produced elicited DTH reactions which were similar to those induced by tuberculin produced by the standard technique. However, under the conditions chosen, the BCG bacilli in the bioreactor cultures formed large bacterial aggregates, impairing exact enumeration of CFU. Yet, measures to reduce the bacterial aggregates were not taken (Nyabenda et al., 1988).
WO 99/49075 discloses a process for microbial conversion of phytosterols to androstenedione and androstadienedione. Said process comprises the growth of mycobacterial cells in polypropylene glycol or silicone. Yet, whereas this process may lead to a reasonable fermentation of phytosterol compositions, said process does not provide for culture conditions which lead to a high percentage of aggregate-free mycobacteria.
All *M*. *bovis* BCG culture conditions described so far have in common, that the bacteria form aggregates of varying size. In addition, all culture conditions lead to a proportion of living to dead bacilli far from optimal. Both problems in turn impair the establishment of reproducible cultures conditions or even the recording of important culture parameters such as numbers of live bacilli/ml. Therefore, correction of culture parameters during growth is almost impossible.

Therefore, the technical problem underlying the present invention was to provide for culture conditions for mycobacteria, in particular for *M*. *bovis* BCG, which allow the reproducible growth of said bacteria, in particular of clump-less cultures comprising a high content of viable mycobacterial cells.

The solution to said technical problem is achieved by providing the embodiment characterized in the claims.

Accordingly, the present invention relates to a culture medium for dispersed mycobacteria comprising at least one detergent and an Antifoam agent, whereby said antifoam agent is a member of the silicone family.

The term "dispersed mycobacterial culture" as employed in the present invention relates to a mycobacterial culture which preferably has at the most 20%, more preferably at the most 10%, more preferably at the most 5% and more preferably at the most 2% aggregated bacilli. The aggregate size is preferably at the most 100 bacteria per aggregate, more preferably at the most 25 bacteria per aggregate, more preferably at the most 10 bacteria per aggregate and more preferably 3 bacteria per aggregate and more preferably at the most 2 bacteria per aggregate. Most preferred are bacterial cultures which are aggregate-/clump-free and comprise only single bacteria or bacteria which are in the process of cell division, i.e. bacteria which are temporarily attached to each other until cell division is completed.
Preferably, dispersed mycobacterial cultures lead to mycobacteria whcih can be determined as "viable units". Said "viable unit" may be determined by methods known in the art which comprise, but are not limited to TVC- (total viable counts) and CFU-(colony forming units) determination. TVC may be determined by fluorescence labeling of viable bacili with Fluorassure^{™} reagent (Chemunex, Ivry sur Seine, France) according to the manufacturers's protocol. Fluorassure^{™} reagent consists of fluorogenic esters which are hydrolyzed by esterases present only in live bacteria, leading to the accumulation of fluorescent product in viable bacterial cells (Breeuwer et al., 1995; Diaper and Edwards, 1994). Fluorescent signals from viable bacteria can be measured with a Chemscan apparatus (Chemunex); see also Breeuwer, Appl. Environm. Microbiol. 61 (1995), 1614-1619 and Diaper, J. Appl. Bacteriol. 77 (1994), 221-228. Preferably, the culture medium provided in this invention leads to a ratio of 100:10, preferably of 100:5, more preferably of 100:2 of viable, preferably single cells to dead cells.

The term "member of the silicone family" relates to silicone polymers known in the art. Said silicone polymers comprise," SiO₂-polymer(s) of variable chain length. Most preferably said silicone polymer is used without emulsifiers.

Preferably, the culture medium described herein, comprises as member of the silicone family, whereby said silicone is in a concentration range of 0.0001 % (v/v) to 10% (v/v). Most preferably, said silicone is in a concentration range of 0.001% (v/v) to 1% (v/v). As documented in the appended examples, a particular preferred concentration for said silicone is 0.015% (v/v). For example, in the experiments described herein, a commercially available silicone was used. In particular, "Antifoam emulsion C^{™}" provided by Sigma-Aldrich (Cat. Number A8011) was employed which comprises as active ingredient 30% aqueous emulsion of "Antifoam A concentrate^{™}, being a 100% active silicone polymer without emulsifiers. When, as shown in the appended examples, said "Antifoam emulsion C^{™}" was employed at 0.05% (v/v) final concentration of silicone was 0.015% (v/v).

Preferably, in accordance with the invention, the culture medium as described above, comprises a detergent selected from the group consisting of Tween 80™, Tween 20^{™}, Triton WR-1339™, Triton X-100^{™}, Triton X-405^{™}, Triton X-114^{™}, Triton N-101^{™}, Triton VG-110^{™}, and Triton XL-80N^{™}. However, further detergents are envisaged, whereby, in particular, said detergent is a detergent which reduces the surface tension of water, which allows the suspension of water-insoluble substances (like the waxes and lipids) present on the surface of mycobacteria, and/or which enables the single cell presence of mycobacteria in the growth medium without formation of aggregates. Most preferably, said detergent is in the concentration of 0.01% (v/v) to 1% (v/v), most preferably, in the concentration 0.3% (v/v).

The culture medium of the present invention may comprise a synthetic medium or a complex medium. Said synthetic medium may be Sauton-medium or Kirchner-medium; as well as Proskauer and Beck medium or Proskauer-Beck medium (Proskauer, Z. Hyg. Infekt. 18 (1894), 128-152). and said complex medium preferably is selected from the group consisting of Dubos-medium, Ungar-medium, 7H9-medium, Middlebrook 7H11-medium and Lowenstein/Lowenstein-Jensen medium and glycine alanine salts broth (GAS), as described by Roberts in "Methods in Microbiology, vol. 25 Immunology of Infection" S.H.E. Kaufmann and D. Kabelitz, eds., Academic Press, San Diego.
However, further media known in the art for growing and/or fermenting mycobacteria may be employed as basic media. Preferably, said media are useful in bioreactor fermentations, in particular in bioreactor of industrial standards. In particular, said bioreactors comprise culture reactors of at least 300 ml, more preferably of at least 500 ml, most preferably of at least 1 liter and most preferably of at least 3 liters. Yet, further bioreactors are envisaged in accordance with this invention, which comprise culturing volumes in the range of 300 ml to 2000 liters. Such bioreactors are known in the art, e.g. the Braun Biostat-E bioreactor (Braun, Melsungen, Germany), and comprise, inter alia, Biostat B-DCU, Biostat B, Biostat CT, Biostat Q, Biostat A, Biostat c-DCU, Biostat DU, Biostat D50, Biostat D100, Biostat D-ASME version, Biostat DL, Biostat D300 and Biostat D500 (all Braun, Melsungen, Germany) bioreactors.

Preferably, the culture medium of the invention is employed for growing/fermenting dispersed mycobacteria selected from the group consisting of M. tuberculosis, M. bovis, M. avium, M. africanum, M. kanasasii, M. intracellulare, M. ulcerans, M. paratuberculosis, M. simiae, M. scrofulaceum, M. szulgai, M. xenopi, M. fortuitum, M. chelonei, M. leprae and M. marinum. Most preferably, said M. tuberculosis is M. tuberculosis H37Rv, M. tuberculsis Erdman or M. tuberculosis CSU93 and said M. bovis is M. bovis BCG, more preferably M. bovis BCG Copenhagen/Copenhagen-1331, M. bovis BCG Pasteur/Pasteur-1173P2, M. bovis BCG Glaxo/Glaxo-1077, M. bovis BCG New York, M. bovis BCG Tokyolfokyo-172, M. bovis BCG Montreal, M. bovis BCG Moreau (Brazil), M. bovis BCG Russian, M. bovis BCG Swedish, M. bovis BCG Tice, M. bovis BCG Beijing, M. bovis BCG Prague, M. bovis BCG Dutch, M. bovis BCG Indonesian, M. bovis BCG Dakar, and most preferably M. bovis BCG Copenhagen/Copenhagen-1331.

In another embodiment, the present invention provides for the use of a culture medium as described herein for the cultivation of mycobacterial strains for vaccination. The dispersed mycobacteria as obtainable with a culture medium as described herein or a method as described herein below are particularly useful for vaccination of mammals, in particular of humans. Said vaccination provides for the elicitation of an immune response in said mammal.

Therefore, the dispersed mycobacteria obtainable by employing the culture media as disclosed herein or by employing the methods provided in this invention may be used to elicit and/or to trigger an immune response in a mammal, preferably in a human.

A vaccination protocol can comprise active or passive immunization, whereby active immunization entails the administration of the dispersed mycobacteria or of proteins, cellular membranes, nucleic acid molecules and the like, antigenic fragments isolated from said dispersed mycobacteria, to the host/patient in an attempt to elicit a protective immune response. Passive immunization entails the transfer of preformed immunoglobulins or derivatives or fragments thereof (e.g., the antibodies, the derivatives or fragments thereof which may be generated by conventional methods against the dispersed mycobacteria grown in the culture medium of the present invention or obtained by methods disclosed herein) to a host/patient. Principles and practice of vaccination and vaccines are known to the skilled artisan, see, for example, in Paul, "Fundamental Immunology" Raven Press, New York (1989) or Morein, "Concepts in Vaccine Development", ed: S.H.E. Kaufmann, Walter de Gruyter, Berlin, New York (1996), 243-264. Typically, vaccines are prepared as injectables, either as liquid solutions or suspensions; solid forms suitable for solution in or suspension in liquid prior to injection also may be prepared. The preparation may be emulsified or the protein may be encapsulated in liposomes. The active immunogenic ingredients often are mixed with pharmacologically acceptable excipients which are compatible with the active ingredient. Suitable excipients include but are not limited to water, saline, dextrose, glycerol, ethanol and the like; combinations of these excipients in various amounts also may be used. The vaccine also may contain small amounts of auxiliary substances such as wetting or emulsifying reagents, pH buffering agents, and/or adjuvants which enhance the effectiveness of the vaccine. For example, such adjuvants can include aluminum compositions, like aluminumhydroxide, aluminumphosphate or aluminumphosphohydroxide (as used in "Gen H-B-Va®" or "DPT-impfstoff Behring"), N-acetyl-muramyl-L-threonyl-D-isoglutamine (thr-DMP), N-acetyl-nomuramyl-L-alanyl-D-isogfulamine (CGP 11687, also referred to as nor-MDP), N-acetylmuramyul-L-alanyl-D-isoglutaminyl-L-alanine-2-(1'2'-dipalmitoyl-sn-glycero-3-hydroxphaosphoryloxy)-ethylamine (CGP 19835A, also referred to as MTP-PE), MF59 and RIBI (MPL + TDM + CWS) in a 2% squalene/Tween-80® emulsion. Additional adjuvants may comprise bacterial toxins like the Escherichia coli heat labile toxin HLT or variants or parts thereof or other immunostimulating toxins like cholera toxin of Vibrio cholerae or listeriolysin or Listeria monocytogenes or variants or parts of these bacterial toxins. Further adjuvants may comprise DNA or oligonucleotides, like, inter alia, CpG-containing motifs (CpG-oligonucleotides; Krieg, Nature 374 (1995), 546-549; Pisetsky, An. Internal. Med. 126 (1997), 169-171).

The vaccines usually are administered by intravenous or intramuscular injection. Additional formulations which are suitable for other modes of administration include suppositories and, in some cases, oral formulations. Further administrations of the vaccines described herein comprise transcutaneous and/or transdermal administration. For suppositories, traditional binders and carriers may include but are not limited to polyalkylene glycols or triglycerides. Oral formulations include such normally employed excipients as, for example, pharmaceutical grades of mannitol, lactose, starch, magnesium stearate, sodium saccharine, cellulose, magnesium carbonate and the like. These compositions may take the form of solutions, suspensions, tablets, pills, capsules, sustained release formulations or powders and contain about 10% to about 95% of active ingredient, preferably about 25% to about 70%.

Vaccines are administered in a way compatible with the dosage formulation, and in such amounts as will be prophylactically and/or therapeutically effective. The quantity to be adminstered generally is in the range of about 5 micrograms to about 250 micrograms of antigen per dose, and depends upon the subject to be dosed, the capacity of the subject's immune system to synthesize antibodies and mount immune responses other than antibody production, and the degree of protection sought. Said antigen may be isolated from the dispersed mycobacteria or standardized mycobacterial culture described herein. Furthermore, isolated dispersed mycobacteria or standardized mycobacterial cultures as described herein may be employed in vaccination protocols. Preferably, 10⁴ to 10⁸ living cells, more preferably 10⁵ to 10⁷ cells, most preferably 10⁶ to 10⁷ living bacterial cells are employed in these protocols. Precise amounts of active ingredient required to be administered also may depend upon the judgment of the practitioner and may be unique to each subject. The vaccine may be given in a single or multiple dose schedule. A multiple dose is one in which a primary course of vaccination may be with one to ten separate doses, followed by other doses given at subsequent time intervals required to maintain and/or to reinforce the immune response, for example, at one to four months for a second dose, and if required by the individual, a subsequent dose(s) after several months. Yet, said doses may also be given at intervals of only one to several days. The dosage regimen also will be determined, at least in part, by the need of the individual, and be dependent upon the practitioner's judgment. It is contemplated that the vaccine containing the immunogenic compounds described herein, i.e. the dispersed mycobacteria, the standardized mycobacterial cultures, or fragments of said dispersed mycobacteria as described herein may be administered in conjunction with other immunoregulatory agents, for example, with immunoglobulins, with cytokines or with molecules which optimize antigen processing, like listeriolysin.

The invention also provides for the use of the culture medium of the invention for the cultivation of mycobacterial strains for cancer immunotherapy or allergy therapy.
It is envisaged that dispersed mycobacteria or standardized cultures of mycobacteria as described herein may be employed for preventing, treating and/or eliviating diseases and disorders which are not primarily caused by mycobacteria. For example, said dispersed mycobacteria or standardized mycobacterial cultures may be employed to suppress allergies or to treat proliferative disorders, like cancer (for example bladder cancer)/tumor growth; see also Erb (1998), Scanga (2000), Kim (1977) or Duda (1995). Further proliferative disorders comprise in this context: melanoma/ malignant melanoma (Henz, 1996); lung cancer (Grant, 1999), adenocarcinoma (Holmang, 2000), renal carcinoma (Nishino, 2000), hepatocarcinoma (Steerenberg, 1991); colon cancer (Habal, 2001), leukemia (Haagenbeck, 1983);in cattle: papilloma and carcinoma (Hill, 1994).

Furthermore, the invention provides the use of the culture medium described herein for the cultivation of mycobacterial strains expressing heterologous proteins. It is, e.g., envisaged that the mycobacterial strains be recombinantly modified and that said recombinantly modified mycobacteria are grown in the culture medium of the present invention in order to obtain dispersed mycobacterial cultures and/or standardized mycobacterial cultures as described herein. These dispersed culture or standardized cultures may be further employed to isolate said heterologously expressed protein/polypeptide.

The mycobacterial strains to be grown in the culture media of the present invention may therefore be a mycobacterial strain comprising heterologous nucleic acid molecules, preferably DNA, which lead to the expression of a heterologous protein and/or substance. Preferably said heterologous protein and/or substance comprises a mycobacterial protein from another strain (e.g. M. tuberculosis protein expressed in M. bovis), a lymphokine, a heterologous bacterial toxin, a heterologous antigen of, inter alia, a pathogenic organism or a tumor antigen. Said heterologous substance may also comprise lipids, glycolipids, saccharides, nucleic acids, glycoproteins or lipoproteins.

Preferably, in the use as described herein said mycobacterial strain is an attenuated strain, however, also "virulent strains" and/or "wildtype strains" may be grown in the culture medium of the present invention and may be used in particular medical settings.

The term "virulent strain", in accordance with the present invention, denotes the capacity of a pathogenic strain of mycobacteria to infect a host and/or to cause disease - defined broadly in terms of severity of symptoms in a host Thus, a "virulent strain" might cause symptoms in a susceptible host, whereas another host might be unaffected by this strain, which can be therefore considered as being an "avirulent strain" in this second host. As used in accordance with the present invention, the term "avirulent strain" denotes strains of mycobacteria which are not capable of inducing infection and/or causing disease in a specific host or in a host species. The term "avirulent strains" denotes furthermore attenuated strains of microorganisms. Said alternated strains and said virulent strains may also be recombinant strains, like, e.g. the *erp*-mutant of *Mycobacterium tuberculosis* (Berthet et al., 1998), a leucine auxotrophic strain of Mycobacterium tuberculosis [Hondalus, (2000). Infect. Immun. *68*, 2888-2898] or a purine auxotrophic strain of Mycobacterium tuberculosis (Jackson et al., 1999).

The mycobacterial strains to be grown in the culture media of the present invention may also be mycobacteria which carry heterologous nucleic acids and which introduce said nucleic acids in vitro or in vivo to a mammalian host cell, whereby, the heterologous nucleic acids comprise a mammalian host cell compatible promoter, operably linked to a structural gene encoding a protein, whereby the protein encoded by the gene can be an antigen or a therapeutic or an immunomodulatory protein.

The invention also relates to a method for the cultivation of dispersed mycobacteria comprising growing said mycobacteria in the culture medium as described herein.

It is preferred in the method of the invention that the culture medium is stirred during the cultivation of said mycobacteria. Said stirring may comprise all stirring methods known in the art and may not only comprise mechanical stirring but also gas-stirring methods.

Yet, in a preferred embodiment said stirring is a mechanical stirring. As shown and documented in the appended example, the present invention provides for a culture medium for culturing dispersed mycobacteria and a method for cultivation of the same, which may also comprise mechanical stirring. This is in clear contrast to the teachings of van Hemert (1971), loc. cit., who described that mechanical stirring is detrimental to the viability of BCG bacilli. Preferably, the mechanical stirring comprises a stirring in a range of 50 to 1000 rpm, more preferably in the range of 350 to 500 rpm. It is of note that in accordance with this invention, the mechanical stirring rate may be modified and changed during the fermentation process/cultivation process described herein.

In a preferred embodiment of the present invention, the oxygen partial pressure (pO₂) is kept above 5% and under 80% saturation. Most preferably, said oxygen partial pressure (pO₂) is kept between 20% and 35% saturation.
As documented in the appended examples, it is preferred that upon each decrease of the oxygen partial pressure (pO₂) to 20% saturation, the pO₂ is raised to 35% saturation. Said increase in pO₂-saturation may be combined with an increase of stirring, e.g. the stirring speed as defined herein above. It is, e.g., envisaged that said stirring speed is increased from 350 rpm to 500 rpm. Said increase in stirring speed may be for a short time, for example for 1 minute, preferably for 2 minutes, more preferably for 3 minutes. As documented herein and in particular in the appended examples, the method of the present invention may also comprise pulsed aeration.

The invention also provides for a method for obtaining a standardized mycobacterial culture comprising the steps of
(a) culturing a potentially mixed mycobacterial culture in the culture medium as defined herein;
(b) isolating a dispersed and/or single mycobacterial cell; and
(c) growing said isolated dispersed and/or single mycobacterial cell in fresh medium.

The term "standardized mycobacterial cell" as employed herein relates to cultures which have been grown from isolated dispersed and/or single mycobacterial cells. Said standardized mycobacterial cultures are particular useful for vaccination protocols, for vaccine efficacy tests, for diagnostic purposes as well as for the preparation of pharmaceutical composition as described herein.

Therefore, the present invention also relates to a pharmaceutical composition comprising dispersed mycobacteria as cultured by the method described herein and/or comprising standardized mycobacterial cultures as obtainable by the method described herein above. As mentioned herein, said dispersed mycobacteria and/or standardized cultures are particularly useful in vaccination protocols, in particular against mycobacterial-induced disease as described herein below and may also be employed in further medical settings.
Therefore, said pharmaceutical composition may be employed in cancer therapy, preferably cancer immuno-therapy or allergy therapy, as explained herein above.

The term "composition", as used in accordance with the present invention, comprises at least one dispersed mycobacterial cell as described herein or a fragment thereof as defined herein and, optionally, further molecules, either alone or in combination, like e.g. molecules which are capable of optimizing antigen processing, cytokines, immunoglobulins, lymphokines or CpG-containing DNA stretches or, optionally, adjuvants, such as Alum or MF59 or other such as 3dmpl, QS21 or mpl. The composition may be in solid, liquid or gaseous form and may be, inter alia, in form of (a) powder(s), (a) tablet(s), (a) solution(s), (a) gel(s) or (an) aerosol(s). In a preferred embodiment, said composition comprises at least one dispersed mycobacterial cell, or at least one cell from a standardized mycobacterial culture described herein and/or isolated fragments, proteins, membranes from said dispersed mycobacterial cultures of standardized cultures combined with at least one, preferably two, preferably three, more preferably four, most preferably five differentially expressed proteins. Differentially expressed proteins, in particular of mycobacteria are known in the art and, inter alia, described in WO 00/44392.

The pharmaceutical composition as described herein above may comprise the herein-defined dispersed mycobacteria or standardized cultures (or cellular fragments of said mycobacteria), either alone or in combination. The pharmaceutical composition of the present invention may be used for effective therapy of infected humans and animals as well as for vaccination purposes and/or prevention purposes in humans and animals.

It is of note that the pharmaceutical composition of the present invention may, additionally, comprise further antigenic determinants or determinants capable of eliciting an immune response or reaction. Said determinants may comprise other differentially expressed polypeptides, like differentially expressed polypeptides as described in WO 00/44392. These further determinants may comprise, but are not limited to proteins/polypeptides which are differentially expressed in M. tuberculosis H37Rv/Erdman as compared to M. bovis BCG. Such proteins comprise Rv3710, Rv1392, Rv0952, Rv2971, Rv0068, Rv0120c, Rv2883c, Rv1463, Rv1856c, Rv2579, Rv3275c, Rv2557, Rv3407, Rv3881c, Rv2449c, Rv0036c, Rv2005c or Rv3676. Functional fragments of said polypeptides capable of eliciting an immunologic reaction or being antigenic fragments, are also envisaged to be comprised, preferably additionally comprised in the (pharmaceutical) composition of this invention. It is understood that also polynucleotides/nucleic acid molecules encoding said differentially expressed polypeptides or functional fragments thereof be comprised in said (pharmaceutical) composition.

The pharmaceutical composition of the present invention may further comprise a pharmaceutically acceptable carrier, excipient and/or diluent. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal, oral, intratracheal, transdermal, transcutaneous, ocular, rectal, vaginal or intrabronchial administration. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Proteinaceous pharmaceutically active matter may be present in amounts between 1 ng and 10 mg per dose; however, doses below or above this exemplary range are envisioned, especially considering the aforementioned factors. Administration of the suitable compositions may be effected by different ways, e.g., by intravenous, intraperitoneal, subcutaneous, intramuscular, topical, oral, intratracheal, transdermal, transcutaneous, ocular, rectal, vaginal or intradermal administration. If the regimen is a continuous infusion, it should also be in the range of 1 µg to 10 mg units per kilogram of body weight per minute, respectively. Progress can be monitored by periodic assessment. The compositions of the invention may be administered locally or systemically. Administration will generally be parenterally, e.g., intravenously. The compositions of the invention may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition of the invention may comprise further agents such as interleukins, interferons and/or CpG-containing DNA stretches, depending on the intended use of the pharmaceutical composition.

For vaccination purposes, the mycobacterial culture can be grown according to the conditions described in the present invention and processed according to Example 6 of the present invention and used for vaccination of humans and animals. Alternatively, the vaccine processing can be done according to the methods presently in use for the BCG vaccine grown as a surface pellicle and described for example in the European Pharmacopoeia, (1997) 3rd edition. Council of Europe, Strasbourg: Typically, after growth of the mycobacterial cultures the bacteria are harvested, e.g. by centrifugation, and the bacilli are resuspended in a suitable solute or medium which can contain stabilizers like sodium glutamate, hemaccel, sucrose, lactose, trehalose, dextrose, glucose, albumin, salts, combinations thereof or other stabilizers. Subsequently, the bacillary mass is filled into appropriate, sealable vials and lyophilized. After lyophilization, the vials are sealed under vacuum or an appropriate gas like nitrogen. The vaccine is stored at temperatures below 8°C until use. The BCG vaccine is sensitive to light and must be protected from light in the container. Upon use, the lyophilized bacilli are resuspended in an appropriate solute like phosphate buffered saline, and injected either parenterally or subcutaneously.

The invention also provides for a method for the production of purified protein derivate(s) (PPD) comprising the steps of
(a) culturing mycobacteria in the inventive culture medium as described herein; and
(b) isolating the PPD.

The isolation of PPD may comprise the inactivation of the mycobacterial culture, for example at 105°C for 1.5 h, the filtration of the inactivated material and the purification of PPD(s), e.g. by ammonium sulfate-precipitation. The precipitates may be further processed, e.g. by neutralizations, steril filtrations and/or lyophilizations. PPD-isolation methods are, inter alia, described in Lindner (1953), Behring Inst. Mitteilungen 27, 165. Purified protein derivatives are in particular useful in diagnostic purposes, e.g. the diagnosis of mycobacterial infection.
To this end, the PPD is applied to an animal or human (e.g. by subcutaneous or intradermal injection) and the immune response elicited is observed. The strength of the immune response allows the diagnosis of mycobacterial infections. Alternatively, the PPD can be used to assess sera or other body fluids of animals or humans for antibodies against PPD antigens, e.g. by ELISA, or Western Blot or other methods known in the art. Furthermore, T cells and B cells specific for PPD antigens may be assessed by T cell and B cell proliferation and/or activation assays or other methods known in the art.

In addition, the present invention provides for the use of the standard mycobacterial culture as obtained by the method described herein above or of the dispersed mycobacteria as cultured by the method described herein or for the preparation of a diagnostic composition for vaccine efficacy testing.

Furthermore, the invention relates to the use of the standardized mycobacterial culture as obtained by the method described herein or use of the dispersed mycobacteria as cultured by the method of the invention for the preparation of a pharmaceutical composition for vaccination against mycobacterial induced disease. Preferably, said mycobacterial-induced diseases comprise tuberculosis, leprosy, skin diseases due to infections with M.chelonei, M. fortitum and M. marinum, buruli ulcer due to M. ulcerans, veterinary infections, like bovine tuberculosis caused e.g. by M. bovis and fish tuberculosis in fish, caused by M. marinum.

Yet, in a further embodiment, the present invention provides for the use of the standardized mycobacterial culture as obtained by the method described herein or use of the dispersed mycobacteria as cultured by the method of the invention for the preparation of a pharmaceutical composition for cancer, immuno therapy or allergy therapy.

In addition, the invention relates to a kit comprising the inventive culture medium described herein. The kit of the present invention is particularly useful in carrying out the method of the invention that has been described herein.

The Figures show:

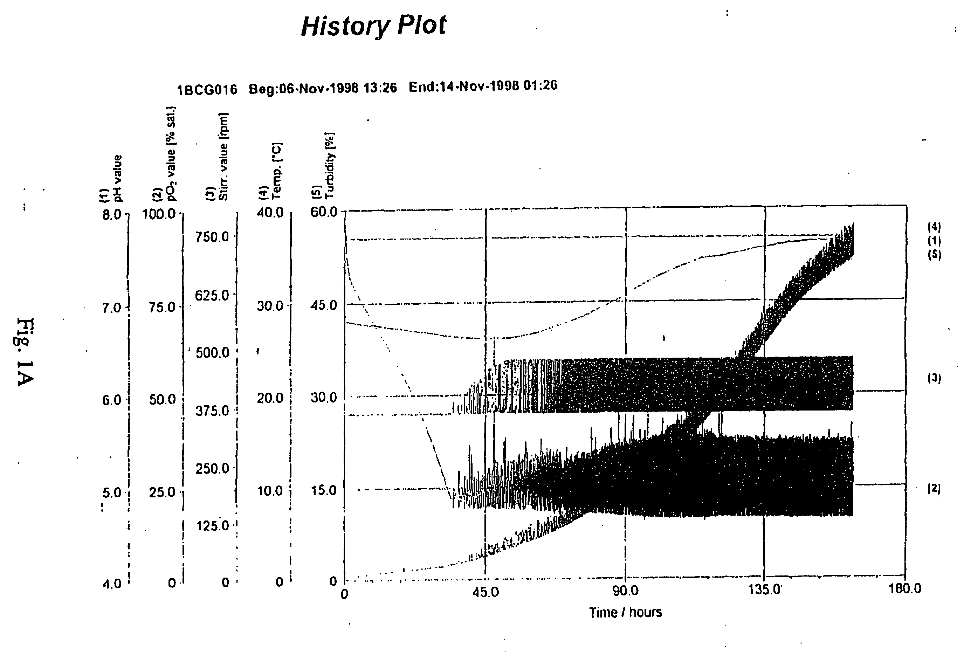

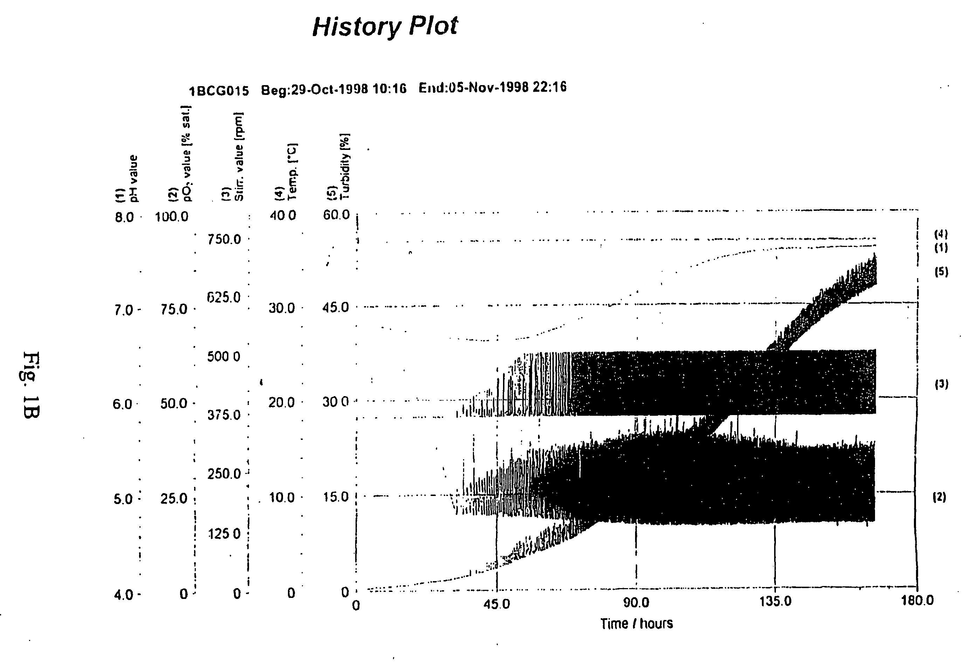

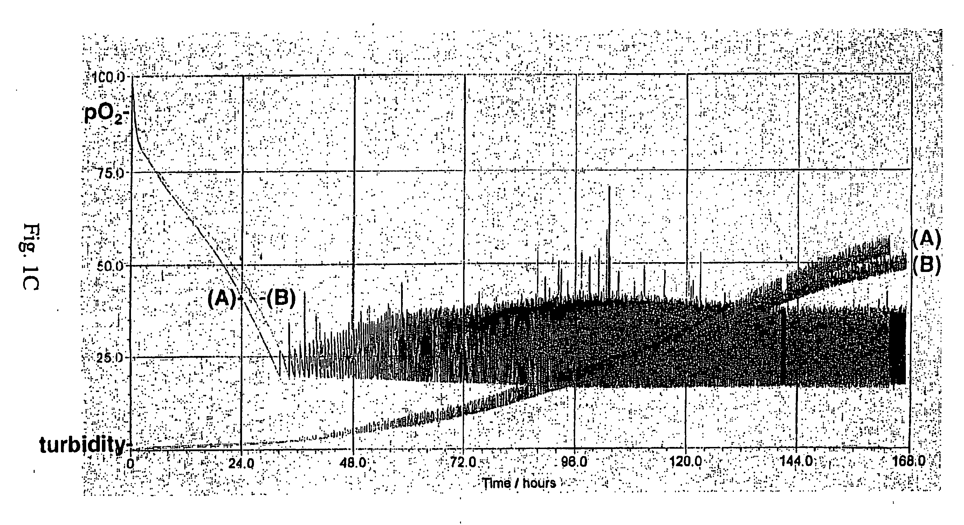

- **Figure 1**: shows the growth parameter recordings of independent homogenous cultures (a and b) of *M. bovis* BCG during cultivation in a bioreactor. Bioreactors were inoculated with 1.0 x 10¹¹ CFU in 21 of Sauton medium (containing 0.3 % Tween 80 and 0.05 % Antifoam emulsion C) and growth parameters were recorded for 7 days. Parameters recorded were changes in turbidity of the cultures, pH changes and changes in pO₂. The temperature was kept at 37°C and rate of stirring was set to 360 rpm. Each time the pO₂ was increased by pulsed aeration, the stirring rate was set to 500 rpm for short periods of time (1 min) and then again reduced to 360 rpm. Figure 1c shows the comparison of turbidity- and pO₂ value- recordings of two independent bioreactor cultures shown in Figure 1a and Figure 1b.

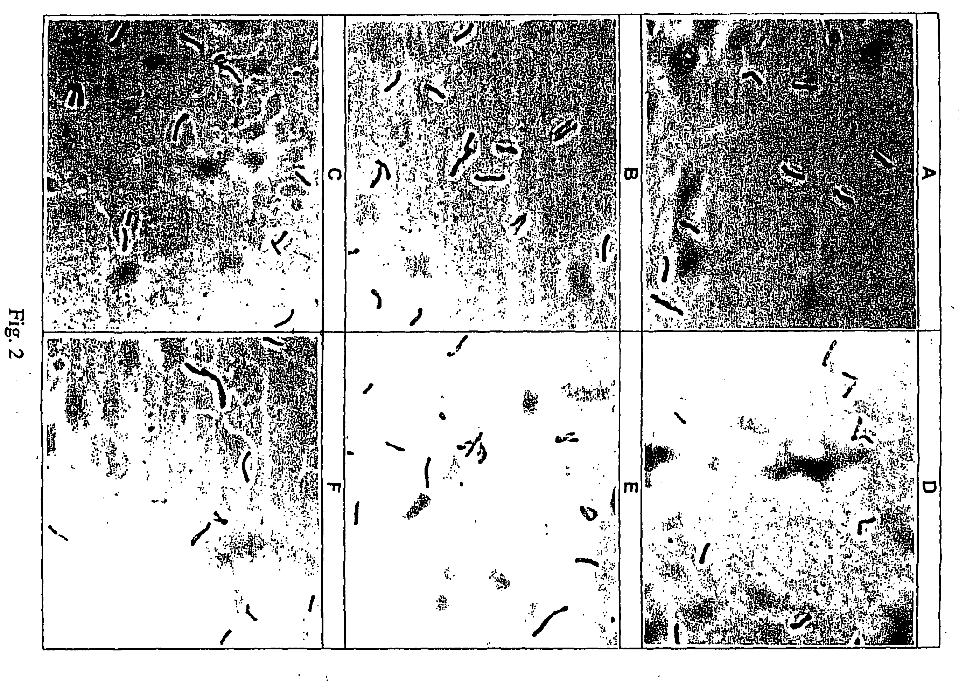

- **Figure 2**: shows phase contrast microscopy images of bacterial suspensions. (a-c) Samples of a typical bioreactor culture were taken at 70 (a), 116 (b) and 164 (c) h post inoculation and examined by phase contrast microscopy. In contrast to the growth conditions of the present invention, the growth conditions described by van Hemert (1971) lead to clumps containing 10 to 100 bacilli per clump as determined by microscopical examination (van Hemert, 1971). (d-f) Bioreactor samples for immunization of mice were taken at 70 (d), 116 (e) and 164 (f) h post inoculation, washed and frozen in PBS, 10% glycerol. After thawing, the bacterial suspensions were examined by phase contrast microscopy.

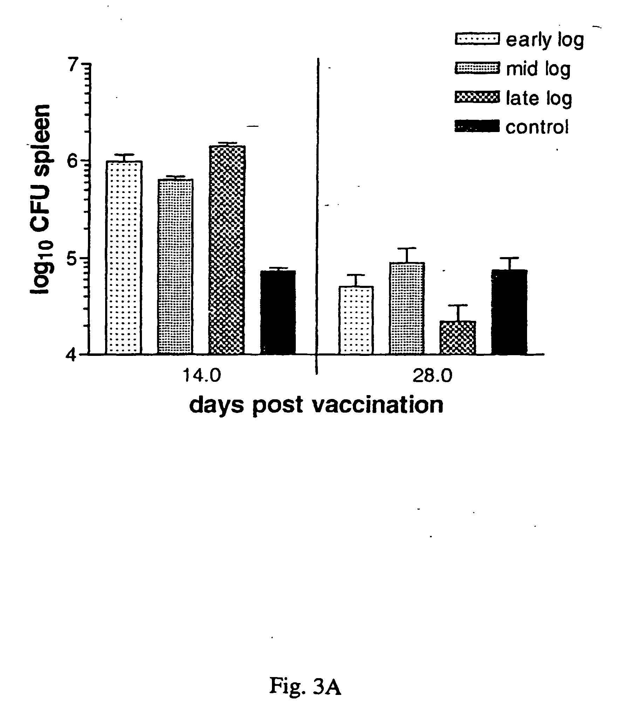

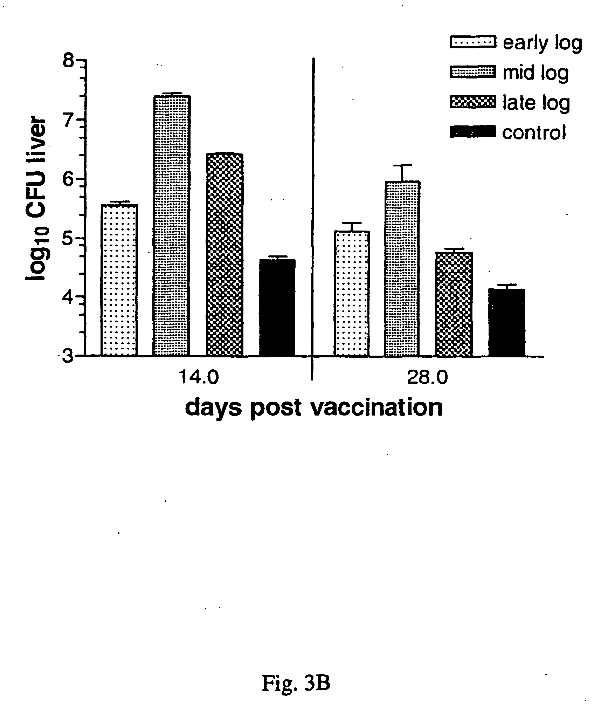

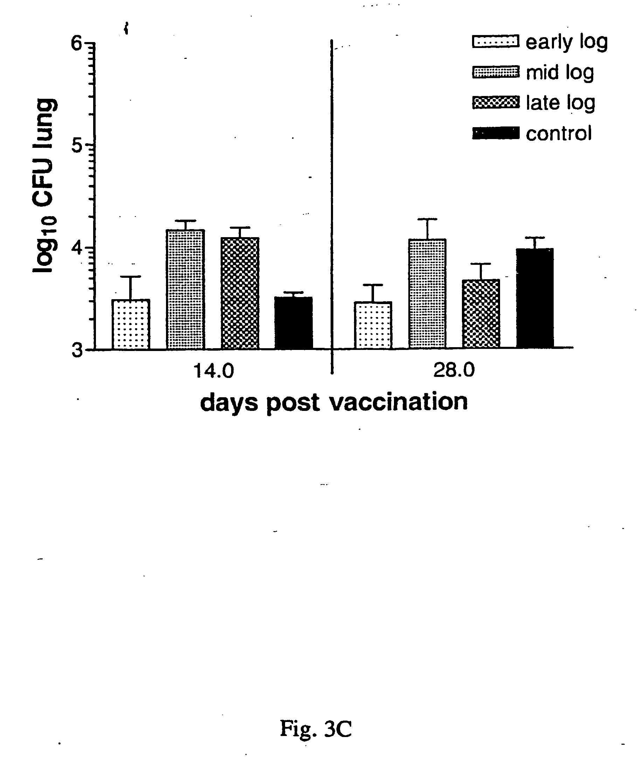

- **Figure 3**: shows the growth and persistence of *M*. *bovis* BCG in mouse organs. From a bioreactor culture grown according to the present invention, *M*. *bovis* BCG Copenhagen were harvested at 70, 116 and 164 h post inoculation (early, middle and late log growth phase) and used for infection of mice. Mice were immunized by i. v. injection of 1 x 10⁶ live CFU of the *M*. *bovis* BCG Copenhagen harvested from the bioreactor culture at 70, 116 and 164 h post inoculation or with 1 × 10⁶ *M. bovis* BCG derived from a classical vaccine production culture grown as a surface pellicle (control). The persistence of the bacilli was determined by plating organ homogenates of spleen, liver and lungs on days 14 and 28 post immunization. Three mice per group were used. *M. bovis* BCG CFU in (A) spleen, (B) liver, (C) lungs are depicted.

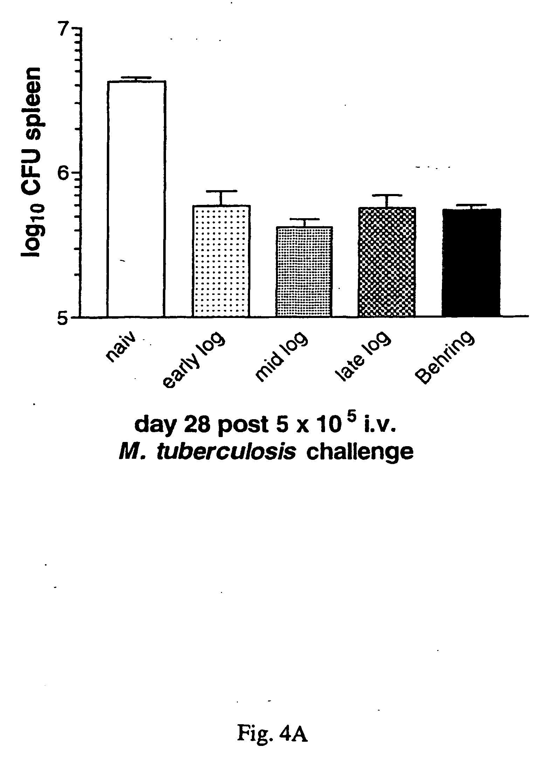

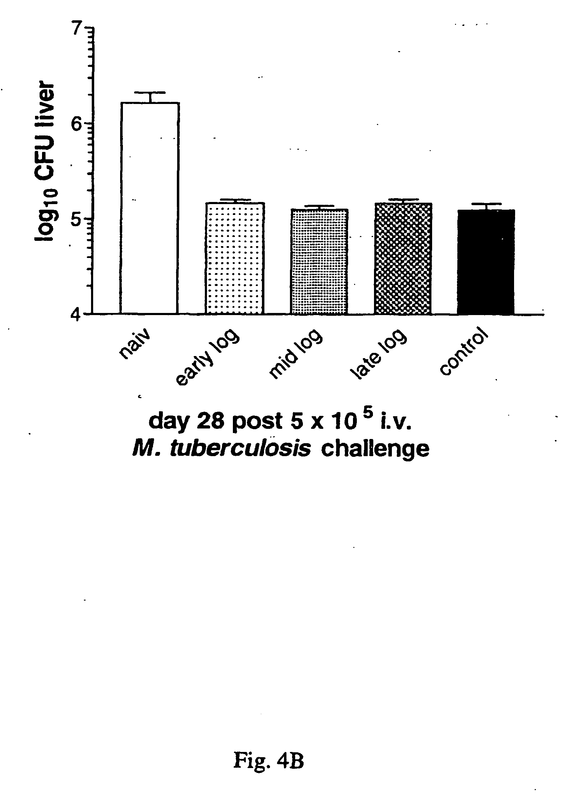

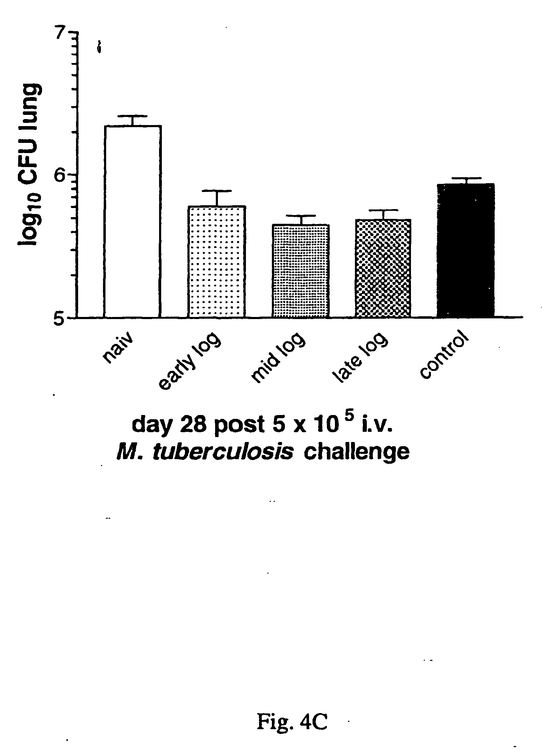

- **Figure 4**: shows the protection of *M*. *bovis* BCG vaccinated mice after *M*. *tuberculosis* H37Rv challenge. Mice immunized with 1 x 10⁶ bioreactor-grown (early, middle and late log phase, respectively) or surface-grown (control) *M*. *bovis* BCG were challenged 57 days post immunization by the i. v. route with 5 × 10⁵ *M*. *tuberculosis* H37Rv. On day 28 post challenge bacterial loads in spleen, liver and lungs were determined by plating serial dilutions of organ homogenates. Naive mice challenged with 5 × 10⁵ CFU *M. tuberculosis* H37Rv served as negative controls. Five mice per group were used. *M. tuberculosis* CFU in (A) spleen, (B) liver and (C) lungs are depicted.

The following Examples illustrate the invention.

### Example 1: Bioreactor cultivation of M. bovis BCG according to the present invention

The strain used for bioreactor cultivation experiments was *M*. *bovis* BCG Copenhagen. *M*. *bovis* BCG Copenhagen was originally obtained from Statens Serum Institute, Copenhagen, Denmark. The cultivation of *M. bovis* BCG Copenhagen in a Braun Biostat-E bioreactor (Braun, Melsungen, Germany) with a 3 L total volume vessel was done in Sauton medium. The Sauton medium was prepared with 5.32 mM L-asparagine, 1.92 mM citric acid, 0.40 mM magnesium sulfate, 0.57 mM dipotassium hydrogen phosphate, 10 mg/l ferric ammonium citrate, 0.35 µM zinc sulfate, 1,0 % (v/v) glycerol. All chemicals were obtained from Riedel de Haen (Germany), pH was adjusted to 7.0, medium was sterile filtered through a 0.2 µm Sealclean filter (Pall Corporation, Port Washington, NY, USA) or autoclaved at 121°C for 30 min. *M*. *bovis* BCG was grown in a Braun Biostat-E bioreactor (Braun, Melsungen, Germany) with a 3 L total volume vessel. Bacteria were cultured in 2 L of Sauton medium. Prior to inoculation, the culture medium was saturated with O₂. The temperature was kept constantly at 37 °C, the pO₂ above 20 % saturation. The stirring rate was 360 rpm. Fermenters were inoculated with 1.0 x 10¹¹ CFU M. bovis BCG. However, experiments with antifoam substances were unsatisfactory, polypropylene glycole as well as a mixture of silicon and liquid paraffin completely inhibited the growth of BCG. Surprisingly, addition of silicone, preferably of Antifoam emulsion C^{™} (Sigma, Germany) to the cultures at 0.05% (v/v) led to growth of the BCG bacteria in the bioreactor and prevented formation of foam in the cultures. The addition of antifoam C did not inhibit the growth of *M*. *bovis* BCG. However, growth of *M. bovis* BCG under these conditions led to bacterial growth at a slow rate and to the formation of bacterial aggregates of up to 1 mm in diameter as well as low reproducibility of the cultures. The size of the bacterial aggregates was dramatically reduced by the addition of 0.01 % (v/v) Tween 80. Optimal dissociation of the bacilli was achieved at 0.3 % (v/v) Tween 80, combined with mechanical stirring and pulsed aeration. Upon a decrease of the pO₂ to 20 % saturation, the pO₂ was raised to about 35 %, combined with an increase of the speed of stirring to 500 rpm for about 1 min. Antifoam emulsion C was added to the cultures at 0.05% (v/v) to prevent formation of foam in the cultures in the presence of detergent.
In contrast to previous findings (van Hemert, 1971 loc. cit.), the mechanical stirring at a high rate (360-500 rpm) was surprisingly not detrimental to the viability of the BCG bacilli. The results of two typical bioreactor culture processes are shown in Figure 1. Each bioreactor run was inoculated with 1.0 x 10¹¹ CFU in 2 L of Sauton medium (containing 0.3 % (v/v) Tween 80 and 0.05 % (v/v) Antifoam emulsion C) and growth parameters were recorded for 7 days. The turbidity of the culture constantly rose until about 160 h of culture when the transition from logarithmic to stationary growth took place. At the time of inoculation, the turbidity of the cultures was 2% (equivalent to OD₆₀₀ₙₘ = 0.13) and it rose to 54% (equivalent to OD₆₀₀ₙₘ = 2.2) at 164 h post inoculation (Fig 1 a and b). The pH of the bioreactor cultures decreased from initially 7.0 to 6.5 during the first 50 h and later increased to 7.6 (Fig 1 a and b). The pO₂ value rapidly fell from 100 % saturation to about 20 % saturation during the first 36 h of bioreactor cultures. It was subsequently kept above 20 % saturation throughout the experiments by pulsed aeration (Fig 1 a and b). Figure 1c shows growth parameters recordings of homogenous cultures of M. bovis BCG during cultivation via a biorector comparison of turbidity and pO2 value recordings of two independent bioreactor cultures as shown in Figure 1 a and Figure 1b.

### Example 2: Bioreactor cultivation of M. bovis BCG according to present invention allows high reproducibility of cultures

The growth of single cell suspensions according to the present invention allows highly reproducible cultivation of mycobacteria, e.g. *M*. *bovis* BCG in bioreactors. Separate bioreactor cultures showed excellent reproducibility with regard to turbidity, pO₂ and pH curves. This in turn supports the adjustment of these parameters to the optimum.

### Example 3: M. bovis BCG grown in bioreactors according to the present invention grow as single cell suspension

At 0, 70, 116 and 164 h after inoculation of a typical bioreactor culture grown according to the present invention, samples were taken to assess bacterial growth. Phase contrast microscopy of the bacteria demonstrated that the *M. bovis* BCG bacilli grew in single cell suspension throughout the bioreactor cultivation process (Fig. 2, a-c). The OD₆₀₀ₙₘ values of the samples taken were approximately 0.1, 0.5, . 1.0 and 2.2, respectively (Table 1).

The complete dissociation of the bacilli is a prerequisite for tight control and standardization of the bioreactor growth process. In contrast to the growth conditions described herein, the growth conditions described by van Hemert (1971) lead to clumps containing 10 to 100 bacilli per clump as determined by microscopical examination (van Hemert, 1971). According to P.A. van Hemert, "...suspensions of B.C.G. always consist of clumps, with the clump size varying in an undefined and uncontrollable way..." (van Hemert, 1971).

### Example 4: M. bovis BCG grown in bioreactors according to the present invention allow exact enumeration of live bacilli

The growth of *M. bovis* BCG as a single cell suspension also enables exact determination of total numbers of viable *M. bovis* BCG bacilli with high reproducibility. At 0, 70, 116 and 164 h after inoculation of a typical bioreactor culture according to the present invention, samples were taken to assess bacterial growth. The OD₆₀₀ₙₘ values of the samples taken were approximately 0.1, 0.5, 1.0 and 2.2, respectively (Table 1). The numbers of live bacilli were determined by determination of total viable counts (TVC) and colony forming units (CFU). TVC were determined by fluorescence labeling of viable bacilli with Fluorassure reagent (Chemunex, Ivry sur Seine, France) according to the manufacturers's protocol. Fluorescent signals from living cells were measured with a Chemscan apparatus (Chemunex). CFU were determined by plating serial dilutions of the bacterial suspensions on Dubos agar plates containing 10 % OADC enrichment and 5 % defibrinated horse blood. Mycobacterial colonies were enumerated after 28 days of incubation at 37°C. The numbers of TVC and CFU per ml were assessed and are shown in Table 1. The bioreactor cultivation process was started with a bacterial density of about 5 x 10⁷ TVC/ml or CFU/ml, and reached about 1.0 x 10⁹/ml when bioreactor growth was terminated at 164 h post inoculation. The numbers of live bacilli were determined as the numbers of CFU or TVC at each time point. In enumerating viable *M*. *bovis* BCG, minimal deviations between numbers of TVC determined by fluorescent labelling of live bacilli and CFU determination by culture on agar plates were found. Deviations of numbers of TVC determined by labelling of live bacilli and of CFU as determined by plating of bacterial suspensions were < 3 %, showing that the cells were viable at the time the samples were taken and were able to form colonies on Dubos agar plates. Furthermore, there were only minimal deviations within each measurement of the number of bacilli, ranging from 1 to maximally 10 % standard deviation for determination of TVC and CFU. The growth of single cell suspensions therefore allows exact quantification of the numbers of BCG bacilli and hence an exact control of bacterial growth in the homogenous bioreactor culture. According to P.A. van Hemert, "...determination of "viable units" (by colony counting on *Löwenstein* medium) appeared to be impossible, because suspensions of B.C.G. always consist of clumps, with the clump size varying in an undefined and uncontrollable way..." (van Hemert, 1971).

### Example 5: Growth of M. bovis BCG according to the present invention gives rise to cultures with high proportion of viable bacilli and allows exact determination of the proportion of live bacilli

The growth of *M*. *bovis* BCG as a single cell suspension described herein also allows exact determination of total numbers of *M*. *bovis* BCG as well as the proportion of viable bacilli with high reproducibility. At 0, 70, 116 and 164 h after inoculation of a typical bioreactor culture grown according to the present invention, samples were taken to assess bacterial growth. The OD₆₀₀ₙₘ values of the samples taken were approximately 0.1, 0.5, 1.0 and 2.2, respectively (Table 1). The numbers of TVC and CFU per ml were assessed and are shown in Table 1. The bioreactor cultivation process was started with a bacterial density of about 5 x 10⁷ TVC/ml or CFU/ml, and reached about 1.0 x 10⁹/ml when bioreactor growth was terminated at 164 h post inoculation. The proportion of live bacilli was determined as the ratio of CFU or TVC to acid fast bacilli (AFB) at each time point. Numbers of AFB were determined by a modified Ziehl-Neelsen-Stain (TB Color, Merck, Darmstadt, Germany) according to the supplier's protocol. At all time points, the numbers of CFU and TVC were above 97 % of the total bacilli determined as AFB. This demonstrates the high viability of the bioreactor grown *M*. *bovis* BCG. Deviations of numbers of TVC determined by labelling of live bacilli and of CFU as determined by plating of bacterial suspensions were < 3 %, showing that the cells were viable at the time the samples were taken and were able to form colonies on Dubos agar plates. Furthermore, there were only minimal deviations within each measurement of the number of bacilli, ranging from 1 to maximally 10 % standard deviation for determination of TVC, CFU and AFB. The growth of single cell suspensions therefore allows the exact determination of the proportion of live bacilli as well as the culture of M. bovis BCG with a high proportion of live bacilli.

### Example 6: Processing of the biorecator-grown M. bovis BCG bacilli grown according to the present invention

Further processing of the bacteria grown as single cell suspension according to the present invention is possible without restrictions. Washing of the cultures with PBS and freezing in PBS/10% glycerol does not cause formation of bacterial aggregates. Samples of a typical bioreactor culture grown according to the present invention were taken at 70, 116 and 164 h post inoculation (early, middle and late logarithmic growth phase, respectively), centrifuged at 4000 x g for 30 min, washed twice with PBS without Ca²⁺, resuspended in PBS containing 10 % glycerol and stored at -70°C. After thawing, the samples showed no aggregation of the bacteria. Microscopic examination demonstrated the preservation of single cell suspensions (Fig 2, d-f). Determination of TVC, CFU and AFB demonstated that only about 1-3 % of the bacteria had lost the capacity of colony formation due to the freezing-thawing process (Table 2). Again, deviations between numbers of TVC and CFU were only minimal.

### Example 7: In vivo replication of biorecator-grown M. bovis BCG bacilli grown according to the present invention

The quality of the bioreactor-grown BCG vaccine grown according to the present invention as determined by the in vivo persistence of the bacilli in mice was equal to that of BCG vaccine produced under the classical production conditions. Replication and persistence of bioreactor culture preparations grown according to the present invention and harvested during early, middle and late logarithmic growth stage (70, 116 and 164 h post inoculation) were analyzed in BALB/c mice. BALB/c mice were bred at the animal facilities at the Bundesinstitut für gesundheitlichen Verbraucherschutz und Veterinärmedizin (BGVV, Berlin, Germany). Animals were kept under specific pathogen-free conditions and fed autoclaved food and water ad libitum. In given experiments, female mice were used at 8 weeks of age. A commercially available *M*. *bovis* BCG Copenhagen vaccine derived from classical vaccine production, grown as a coherent pellicle, served as control. Animals were infected by i. v. injection of 1 x 10⁶ CFU *M*. *bovis* BCG, either of the bacteria grown according to the present invention (and harvested at early, middle and late logarithmic growth stage) or of the bacteria grown under the classical conditions. Growth and persistence were determined on days 14 and 28 post immunization in three mice per group at each time point by plating organ homogenates of spleen, liver and lungs on Middlebrook 7H11 agar supplemented with OADC enrichment 1339 (Fig. 3). On day 14 post immunization, bacterial numbers in the spleen were one order of magnitude higher for all bioreactor cultures (grown according to the present invention) compared to the control (Fig. 3a). The numbers of bioreactor-grown bacilli were rapidly reduced by day 28 post immunization to the value of the classical vaccine, which persisted at unchanged numbers between day 14 and 28. In the livers of vaccinated animals, the numbers of CFU were again reduced in the case of the classical vaccine in comparison to the bioreactor-grown vaccine preparations at day 14 (Fig 3b). Mice immunized with bacilli harvested during the middle logarithmic culture showed higher CFU compared to bacilli from early logarithmic culture in liver. On day 28, the bacilli from middle logarithmic bioreactor culture still showed a enhanced persistence compared to the control. Similar results were obtained when bacterial numbers in the lungs were determined (Fig 3c). The middle and late logarithmic bioreactor cultures exhibited higher numbers of CFU than early logarithmic bioreactor culture or classical vaccine on day 14 post immunization. On day 28, only the numbers of bacilli from the late logarithmic bioreactor culture were reduced, the early and middle logarithmic culture persisted without reduction, while the classical vaccine even exhibited growth to some extent. The bioreactor cultures were slightly more virulent and persistent at an initial stage but were cleared to almost the same extent as the classical vaccine at later time points.
Addition of Tween 80 as dispersing agent to the culture medium did surprisingly not abolish or impair the in vivo growth or persistence of the BCG bacteria grown according to the present invention. Previously, Tween 80 had been found to reduce the in vivo growth of mycobacteria (Bloch and Noll, 1953).

### Example 8: Immunogenicity of M. bovis BCG bacilli grown in biorecators according to the present invention

The quality of the bioreactor-grown BCG vaccine grown by the method described herein as determined by the protective efficacy in mice against a challenge with virulent *M. tuberculosis* was equal to that of BCG vaccine produced under the classical production conditions. Immunogenicity and protective efficacy of bioreactor culture preparations grown as described herein and harvested during early, middle and late logarithmic growth stage (70, 116 and 164 h post inoculation) were analyzed in BALB/c mice. A commercially available *M. bovis* BCG Copenhagen vaccine derived from classical vaccine production, grown as a coherent pellicle, served as control. Animals were immunized by i. v. injection of 1 x 10⁶ CFU *M. bovis* BCG either of the bacteria grown according to the present invention (and harvested at early, middle and late logarithmic growth stage) or of the bacteria grown under the classical conditions. The protective efficacy of all vaccine preparations was tested by i.v. challenge with virulent *M*. *tuberculosis* H37Rv. *M. tuberculosis* H37Rv is a human lung isolate from 1905 and was purchased from the American Type Culture Collection (ATCC), Manassas, USA. Before challenge infection, mice were cured from *M*. *bovis* BCG by antibiotic treatment. All animals underwent a 10-day course of chemotherapy provided in their drinking water with 100 mg/ml rifampicin and 200 mg/ml isoniazide between days 29 and 43 post vaccination. Curing from BCG infection was verified by CFU analysis on Middiebrook 7H11 agar and mice were shown to be BCG free before challenge. On day 56 post vaccination, mice were challenged by i. v. injection of 5 × 10⁵ CFU *M. tuberculosis* H37Rv. On day 28 after H37Rv challenge, bacterial loads of 5 mice per group were determined as CFU in spleen, liver and lungs by plating serial dilutions of organ homogenates on Middlebrook 7H11 agar. Protection was determined as bacterial CFU in lungs, spleens and livers of infected mice four weeks post challenge infection. While the vaccine preparations exhibited differences in their persistence in the mouse, their protective efficacy after i. v. challenge was almost identical with only minimal deviations. On day 28 post challenge with *M*. *tuberculosis,* the numbers of tubercle bacilli were reduced by approximately one order of magnitude in spleen, liver and lungs of vaccinated animals in comparison to naive mice (Fig 4,a-c). Interestingly, the growth phase in the bioreactor culture grown according to the present invention does not seem to have a major impact on the protective efficacy of a given vaccine preparation. In our studies bacilli from early , middle and late logarithmic growth phase grown according to the present invention elicited comparable protection against a virulent *M. tuberculosis* challenge.

### Example 9: Production of a vaccine against infection with M. tuberculosis with M. bovis BCG bacilli

*M. bovis* BCG were grown in a bioreactor as described herein. The bacilli were harvested during early, middle and late logarithmic growth stage (70, 116 and 164 h post inoculation), centrifuged at 4000 x g for 30 min, washed twice with PBS without Ca²⁺, resuspended in PBS containing 10 % glycerol and stored at -70°C.

After thawing, the samples showed no aggregation of the bacteria. Microscopic examination demonstrated the preservation of single cell suspensions (Fig 2, d-f). The bacteria were again cemtrifuged and washed with PBS without Ca²⁺ and resuspended in PBS without Ca²⁺ directly before vaccination of mice. A commercially available *M*. *bovis* BCG Copenhagen vaccine derived from classical vaccine production, grown as a coherent pellicle and resuspended in PBS without Ca²⁺, served as control. BALB/c mice were vaccinated by i. v. injection of 1 x 10⁶ CFU *M. bovis* BCG either of the bacteria grown as described herein above (and harvested at early, middle and late logarithmic growth stage) or of the bacteria grown under the classical conditions. The protective efficacy of all vaccine preparations was tested by i.v. challenge with virulent *M. tuberculosis* H37Rv. M. *tuberculosis* H37Rv is a human lung isolate from 1905 and was purchased from the American Type Culture Collection (ATCC), Manassas, USA. Before challenge infection, mice were cured from *M. bovis* BCG by antibiotic treatment. All animals underwent a 10-day course of chemotherapy provided in their drinking water with 100 mg/ml rifampicin and 200 mg/ml isoniazide between days 29 and 43 post vaccination. Curing from the BCG vaccine infection was verified by CFU analysis on Middlebrook 7H11 agar and mice were shown to be BCG free before challenge. On day 56 post vaccination, mice were challenged by i. v. injection of 5 × 10⁵ CFU *M. tuberculosis* H37Rv. On day 28 after H37Rv challenge, bacterial loads of 5 mice per group were determined as CFU in spleen, liver and lungs by plating serial dilutions of organ homogenates on Middlebrook 7H11 agar. Protection was determined as bacterial CFU in lungs, spleens and livers of infected mice four weeks post challenge infection. While the vaccine preparations exhibited differences in their persistence in the mouse, their protective efficacy after i. v. challenge was almost identical with only minimal deviations. On day 28 post challenge with *M. tuberculosis,* the numbers of tubercle bacilli were reduced by approximately one order of magnitude in spleen, liver and lungs of vaccinated animals in comparison to naive mice (Fig 4,a-c). Interestingly, the growth phase in the bioreactor culture grown according to the present invention does not seem to have a major impact on the protective efficacy of a given vaccine preparation. In our studies bacilli from early , middle and late logarithmic growth phase grown according to the present invention elicited comparable protection against a virulent *M. tuberculosis* challenge and hence represent a suitable vaccine for protection against infection with *M*. *tuberculosis.*

### Tables

**Table 1: Bacterial growth during a typical bioreactor run.**

| Time [h] | Growth parameters | | | |
|---|---|---|---|---|
| | OD₆₀₀ₙₘ | TVC/ml | CFU/ml | AFB/ml |
| 0 | 0.10 +/- 0.01 | 4.95 x 10⁷ +/- 0.06 x 10⁷ | 4.87 x 10⁷ +/- 0.09 x 10⁷ | 5.02 x 10⁷ +/- 0.07 x 10⁷ |
| 70 | 0.51 +/- 0.05 | 2.50 x 10⁸ +/- 0.04 x 10⁸ | 2.45 x 10⁸ +/- 0.12 x 10⁸ | 2-52 x 10⁸ +/- 0.11 x 10⁸ |
| 116 | 1.02 +/- 0.04 | 4.96 x 10⁸ +/- 0.05 x 10⁸ | 5.04 x 10⁸ +/- 0.11 x 10⁸ | 5.10 x 10⁸ +/- 0.09 x 10⁸ |
| 164 | 2.19 +/- 0.08 | 1.01 x 10⁹ +/- 0.03 x 10⁹ | 1.02 x 10⁹ +/- 0.09 x 10⁹ | 1.04 x 10⁶ +/- 0.10 x 108⁹ |

Samples were taken after inoculation of the bioreactor at 0, 70, 116 and 164 h post inoculation. Bacterial growth was determined by measurement of the optical density at OD₆₀₀ₙₘ, numbers of CFU by plating serial dilutions on Dubos agar plates, TVC were determined by labelling viable bacteria with Fluorassure and detection with the Chemscan apparatus and numbers of AFB by a modified Ziehl-Neelsen stain. All numbers were determined in triplicate.

**Table 2: Assessment of bacterial numbers in stocks used for immunization of mice.**

| Time [h] | Growth parameters | | |
|---|---|---|---|
| | TVC/ml | CFU/ml | AFB/ml |
| 70 | 1.06 x 10⁸ +/- 0.04 x 10⁸ | 1.04 x 10⁸ +/- 0.06 x 10⁸ | 1.07 x 10⁸ +/- 0.09 x 10⁸ |
| 116 | 0.74 x 10⁸ +/- 0.03 x 10⁸ | 0.75 x 10⁸ +/- 0.07 x 10⁸ | 0.77 x 10⁸ +/- 0.04 x 10⁸ |
| 164 | 0.86 x 10⁸ +/- 0.05 x 10⁸ | 0.84 x 10⁸ +/- 0.09 x 10⁸ | 0.87 x 10⁸ +/- 0.06 x 10⁸ |

Bacterial samples were taken at 70, 116 and 164 h of a typical bioreactor run, washed twice with PBS and frozen at -70°C in PBS/10% glycerol. After thawing, TVC were determined by labelling viable bacteria with Fluorassure and detection with the Chemscan apparatus, the CFU were determined by plating serial dilutions on Dubos agar plates and the AFB by a modified Ziehl-Neelsen stain. All numbers were determined in triplicate.

### References

Abomoelak, Infect Immun 67 (1999), 5100-5105
Andersen, Infect. Immun. 62 (1994), 2536-2544
Baldwin, Infect. Immun. 66 (1998), 2951-2959
Berthet, Science 282 (1998), 759-762
Bloch, J. Exp. Med. 97 (1953), 1-6.
Bloom, (1994) The BCG experience:implications for future vaccines against tuberculosis. In: Bloom, B.R. (Ed.), Tuberculosis: Pathogenesis, Protection and Control, American Society for Microbiology, Washington, DC pp. 531-557.
Bloom, Science 257 (1992), 1055-1064.
Brandt, Infect Immun 68 (2000), 791-795
Bunch-Kristensen, J. Biol. Stand. 5 (1977), 159-161.
Burkhardt, (ed.) (1992). Mikrobiologische Diagnostik. G. Thieme Verlag, Stuttgart Calmette, (1927) La vaccination Preventive Contre la Tuberculose. Masson et Cie., Paris.
Calmette, Compt. R. Acad. Sci. 149 (1909); 716-720.
Chambers, Infect Immun 68 (2000), 7094-7099
Clemens, JAMA 249 (1983), 2362-2369
Colditz, JAMA 271 (1994), 698-702.
Comstock, Controlled Clin. Trials 15 (1994), 247-276.
Connell, Proc Natl Acad Sci U S A 90 (1993), 11473-11477
Dubos, J. Exp. Med. 91 (1950), 261-268.
Duda, Ann Surg Oncol 2 (1995), 542-549
Erb, J. Exp. Med. 187 (1998), 561-569
Fenner, J. Exp. Med. 91 (1950), 269-284.
Fine, Lancet 346 (1995), 1339-1345.
Gheorghiu, Dev Biol Stand. 58 (1986), 191-205.
Gheorghiu, J. Biol. Stand. 15 (1988), 15-26.
Grant, Clin. Cancer Res. 5 (1999), 1319-1323
Guleria, Nat Med 3 (1996), 334-337
Hagenbeek, Leuk. Res. 7 (1983), 547-555
Hayward, Vaccine 17 (1999), 1272-1281
van Hemert, (1971) BCG vaccine. In: van Hemert, P.A., (Ed.) Vaccine production as a unit process, Leiden, pp. 110-116.
Henz, Detrmatology 193 (1996), 105-109
Hernandez-Pando, Immunology 100 (2000), 391-398
Hess, Proc Natl Acad Sci U S A 95 (1998), 5299-5304
Hill, Cancer Immunol. Immunother 39 (1994), 49-52
Holmang, Scand. J. Urol. Nephrol. 34 (2000), 141-143
Horwitz, Proc Natl Acad Sci U S A97 (2000), 13853-13858
Hubbard, Clin. Exp. Immunol. 87 (1992), 94-98
Jabbar, Vaccine 18 (2000), 2444-2453
Jackson, Infect Immun 67 (1999), 2867-2873
Jensen, Zbl. Bakt. Abt. I Orig. 125 (1932), 222.
Kaufmann, Nat Med. 6 (2000), 955-960.
Kaufmann, PCT/EP 98/05109 (2000)
Kim, Appl Environ Microbiol 34 (1977), 495-499.
Kirchner, Zbl. Bakt.. 1 Orig. 124 (1932), 403
Lagranderie, Vaccine 11 (1993), 1283-1290
Lefford, Immunol. 14 (1974), 417-428
Leung, Virology 268 (2000), 94-103
Levy, Adv. Tuberc. Res. 16 (1968), 63-176
Mahairas, J. Bact. 178 (1996), 1274-1282
Matsumoto, Vaccine 18 (1999), 832-834
Milstien, Bull World Health Organ 68 (1990), 93-108
Nishino, BJU Int. 85 (2000), 799-801
Nyabenda, J Biol Stand 16 (1988), 259-267
Ohara, Vaccine 18 (2000), 1294-1297
Orme, Trends Microbiol 3 (1995), 401-404
Orme, Mol Med Today 5 (1999), 487-492
Pal, Infect. Immun. 60 (1992), 4781-4792
Ravn, J. Immunol. 158 (1997), 1949-1955
Roberts, Immunology 85 (1995), 502-508
Roche, Trends Microbiol. 3 (1995), 397-401.
Rodrigues, Trans. R. Soc. Trop. Med. Hyg. 84 (1990), 739-744
Rodrigues, Int. J. Epidemiol. 22 (1993), 1154-1158.
Scanga, Drugs 59 (2000), 1217-1221
Silver, Immun. 66 (1998), 1190-1199
Sirks, Symp. Series Immunobiol. Stand. 17 (1971), 163-168.
Steerenberg, In Vivo 5 (1991), 655-661
Stover, Nature 351 (1991), 456-460
Stover, J Exp Med 178 (1993),197-209
Streit, Exp Parasitol 94 (2000), 33-41
Ungar, Br. Med. J. 11 (1962), 1086-1089.
Wangoo, Clin. Exp. Immunol. 119 (2000), 92-98
World Health Organization, Bull. W.H.O. 57 (1979), 810-827.
Zhu, J. Infect. Dis. 176 (1997), 1445-1453.

## Claims

1. A culture medium for dispersed mycobacteria comprising at least one detergent and an antifoam agent, whereby said antifoam agent is a silicone polymer.

2. Culture medium of claim 1, wherein said silicone is in a concentration range of 0.0001% (v/v) to 10% (v/v).

3. Culture medium of claim 1 or 2, wherein said silicone is in a concentration range of 0.001% (v/v) to 1% (v/v).

4. Culture medium of any one of claims 1 to 3, wherein said detergent is selected from the group consisting of Tween 80™, Tween 20^{™}, Triton WR-1339™, Triton X-100^{™}, Triton X-405^{™}, Triton X-114^{™}, Triton N-101^{™}, Triton VG-110^{™} and Triton XL-80N^{™}.

5. Culture medium of any one of claims 1 to 4, wherein said detergent is in the concentration of 0.01% (v/v) to 1% (v/v).

6. Culture medium of claim 5, wherein said detergent is in the concentration of 0.3% (v/v).

7. Culture medium of any one of claims 1 to 6, wherein said medium comprises a synthetic medium or a complex medium.

8. Culture medium of claim 7, wherein said synthetic medium is Sauton-medium, Kirchner-medium or Proskauer-Beck-medium.

9. Culture medium of claim 7, wherein said complex medium is Dubos-medium, Ungar-medium, Middlebrook 7H9-medium, Middlebrook 7H11-medium, Löwenstein/Löwenstein-Jensen medium or glycine-alanine salts broth (GAS).

10. Culture medium of any one of claims 1 to 9, wherein said mycobacteria are selected from the group consisting of M. tuberculosis, M. bovis, M. avium, M. africanum, M. kanasasii, M. intracellulare, M. ulcerans, M. paratuberculosis, M. simiae, M. scrofulaceum, M. szulgai, M. xenopi, M. fortuitum, M. chelonei, M. leprae and M. marinum.

11. Culture medium of claim 10, wherein said M. tuberculosis is M. tuberculosis H37Rv, M. tuberculosis Erdman or M. tuberculosis CSU93 and said M. bovis is M. bovis BCG.

12. Use of a culture medium of any one of claims 1 to 11 for the cultivation of mycobacterial strains for vaccination.

13. Use of the culture medium of any one of claims 1 to 11 for the cultivation of mycobacterial strains for cancer immunotherapy or allergy therapy.

14. Use of the culture medium of any one of claims 1 to 11 for the cultivation of mycobacterial strains expressing heterologous proteins.

15. Use of any one of claims 12 to 14, wherein said mycobacterial strain is a attenuated strain.

16. A method for the cultivation of dispersed mycobacteria comprising growing said mycobacteria in the culture medium of any one of claims 1 to 11.

17. The method of claim 16, wherein the culture medium is stirred during the cultivation of said mycobacteria.

18. The method of claim 17, wherein said stirring is a mechanical stirring.

19. The method of claim 17 or 18, whereby said stirring comprises a stirring in a range of 50 to 1000 rpm.

20. The method of claim 19, whereby said stirring comprises a stirring at 360 to 500 rpm.

21. The method of any one of claims 17 to 20, whereby the oxygen partial pressure (pO₂) is kept above 5% and under 80% saturation.

22. The method of claim 21, whereby said oxygen partial pressure (pO₂) is kept between 20% and 35% saturation.

23. A method for obtaining a standardized mycobacterial culture comprising the steps of
(a) culturing a potentially mixed mycobacterial culture in the medium as defined in any one of claims 1 to 11;
(b) isolating a dispersed and/or single mycobacterial cell; and
(c) growing said isolated dispersed and/or single mycobacterial cell in fresh medium.

24. A method for the production of (a) purified protein derivate(s) (PPD) comprising the steps of
(a) culturing mycobacteria in the culture medium of any one of claims 1 to 11; and
(b) isolating the PPD.

25. Use of the standardized mycobacterial culture as obtained by the method of claim 23 or use of the dispersed mycobacteria as cultured by the method of any one of claims 16 to 22 for the preparation of a diagnostic composition for vaccine efficacy testing.

26. Use of the standardized mycobacterial culture as obtained by the method of claim 23 or use of the dispersed mycobacteria as cultured by the method of any one of claims 16 to 22 for the preparation of a pharmaceutical composition for vaccination against mycobacterial induced disease.

27. Use of the standardized mycobacterial culture as obtained by the method of claim 23 or use of the dispersed mycobacteria as cultured by the method of any one of claims 16 to 22 for the preparation of a pharmaceutical composition for cancer immuno therapy or allergy therapy.

28. The use of claim 26, whereby said mycobacterial induced disease comprise tuberculosis, leprosy or skin diseases.

29. Kit comprising the culture medium of any one of claims 1 to 11.

## Patentansprüche

1. Kulturmedium für dispergierte Mycobakterien, umfassend mindestens ein Detergens und ein Antischaummittel, wobei das Antischaummittel ein Silikonpolymer ist.

2. Kulturmedium nach Anspruch 1, wobei das Silikon in einem Konzentrationsbereich von 0,0001 % (v/v) bis 10 % (v/v) vorliegt.

3. Kulturmedium nach Anspruch 1 oder 2, wobei das Silikon in einem Konzentrationsbereich von 0,001 % (v/v) bis 1 % (v/v) vorliegt.

4. Kulturmedium nach einem der Ansprüche 1 bis 3, wobei das Detergens ausgewählt ist aus der Gruppe bestehend aus Tween 80^{™}, Tween 20^{™}, Triton WR-1339^{™}, Triton X-100^{™}, Triton X-405^{™}, Triton X-114^{™}, Triton N-101^{™}, Triton VG-110^{™} und Triton XL-80N^{™}_{.}

5. Kulturmedium nach einem der Ansprüche 1 bis 4, wobei das Detergens in einer Konzentration von 0,01 % (v/v) bis 1 % (v/v) vorliegt.

6. Kulturmedium nach Anspruch 5, wobei das Detergens in einer Konzentration von 0,3 % (v/v) vorliegt.

7. Kulturmedium nach einem der Ansprüche 1 bis 6, wobei das Medium ein synthetisches Medium oder ein komplexes Medium umfasst.

8. Kulturmedium nach Anspruch 7, wobei das synthetische Medium Sauton-Medium, Kirchner-Medium oder Proskauer-Beck-Medium ist.

9. Kulturmedium nach Anspruch 7, wobei das komplexe Medium Dubos-Medium, Ungar-Medium, Middlebrook 7H9-Medium, Middlebrook 7H11-Medium, Löwenstein/Löwenstein-Jensen-Medium oder Glycin-Alanin-Salzbrühe (GAS) ist.

10. Kulturmedium nach einem der Ansprüche 1 bis 9, wobei die Mycobakterien ausgewählt sind aus der Gruppe bestehend aus M. tuberculosis, M. bovis, M. avium, M. africanum, M. kanasasii, M. intracellulare, M. ulcerans, M. paratuberculosis, M. simiae, M. scrofulaceum, M. szulgai, M. xenopi, M. fortuitum, M. chelonei, M. leprae und M. marinum.

11. Kulturmedium nach Anspruch 10, wobei M. tuberculosis M. tuberculosis H37Rv, M. tuberculosis Erdman oder M. tuberculosis CSU93 ist und wobei M. bovis M. bovis BCG ist.

12. Verwendung eines Kulturmediums nach einem der Ansprüche 1 bis 11 für die Züchtung von mycobakteriellen Stämmen zur Impfung.

13. Verwendung des Kulturmediums nach einem der Ansprüche 1 bis 11 für die Züchtung von mycobakteriellen Stämmen zur Krebsimmuntherapie oder Allergietherapie.

14. Verwendung des Kulturmediums nach einem der Ansprüche 1 bis 11 für die Züchtung von mycobakteriellen Stämmen, die heterologe Proteine exprimieren.

15. Verwendung nach einem der Ansprüche 12 bis 14, wobei der mycobakterielle Stamm ein attenuierter Stamm ist.

16. Verfahren für die Züchtung von dispergierten Mycobakterien, umfassend das Züchten der Mycobakterien in dem Kulturmedium nach einem der Ansprüche 1 bis 11.

17. Verfahren nach Anspruch 16, wobei das Kulturmedium während der Züchtung der Mycobakterien gerührt wird.

18. Verfahren nach Anspruch 17, wobei das Rühren ein mechanisches Rühren ist.

19. Verfahren nach Anspruch 17 oder 18, wobei das Rühren ein Rühren in einem Bereich von 50 bis 1000 Upm umfasst.

20. Verfahren nach Anspruch 19, wobei das Rühren ein Rühren bei 360 bis 500 Upm umfasst.

21. Verfahren nach einem der Ansprüche 17 bis 20, wobei der Sauerstoffpartialdruck (pO₂) bei über 5 % und unter 80 % Sättigung gehalten wird.

22. Verfahren nach Anspruch 21, wobei der Sauerstoffpartialdruck (pO₂) zwischen 20 % und 35 % Sättigung gehalten wird.

23. Verfahren zum Erhalten einer standardisierten mycobakteriellen Kultur, umfassend die Schritte
(a) Züchten einer möglicherweise gemischten mycobakteriellen Kultur in dem wie in einem der Ansprüche 1 bis 11 definierten Medium;
(b) Isolieren einer dispergierten und/oder einzelnen mycobakteriellen Zelle; und
(c) Züchten der isolierten dispergierten und/oder einzelnen mycobakteriellen Zelle in frischem Medium.

24. Verfahren für das Erzeugen (eines) gereinigten/r Proteinabkömmlings/e (PPD), umfassend die Schritte
(a) Züchten von Mycobakterien in dem Kulturmedium nach einem der Ansprüche 1 bis 11; und
(b) Isolieren der PPD.

25. Verwendung der standardisierten mycobakteriellen Kultur wie durch das Verfahren nach Anspruch 23 erhalten oder Verwendung der dispergierten Mycobakterien wie durch das Verfahren nach einem der Ansprüche 16 bis 22 gezüchtet für die Herstellung einer diagnostischen Zusammensetzung zum Testen der Wirksamkeit eines Impfstoffs.

26. Verwendung der standardisierten mycobakteriellen Kultur wie durch das Verfahren nach Anspruch 23 erhalten oder Verwendung der dispergierten Mycobakterien wie durch das Verfahren nach einem der Ansprüche 16 bis 22 gezüchtet zur Herstellung eines Arzneimittels für die Impfung gegen eine durch Mycobakterien hervorgerufene Krankheit.

27. Verwendung der standardisierten mycobakteriellen Kultur wie durch das Verfahren nach Anspruch 23 erhalten oder Verwendung der dispergierten Mycobakterien wie durch das Verfahren nach einem der Ansprüche 16 bis 22 gezüchtet zur Herstellung eines Arzneimittels zur Krebsimmuntherapie oder Allergietherapie.

28. Verwendung nach Anspruch 26, wobei die durch Mycobakterien hervorgerufene Krankheit Tuberkulose, Lepra oder Hautkrankheiten umfasst.

29. Kit umfassend das Kulturmedium nach einem der Ansprüche 1 bis 11.

## Revendications

1. Milieu de culture pour des mycobactéries dispersées comprenant au moins un détergent et un agent antimousse, lequel agent antimousse est un polymère de silicone.

2. Milieu de culture selon la revendication 1, dans lequel ladite silicone est dans un domaine de concentration de 0,0001 % (v/v) à 10 % (v/v).

3. Milieu de culture selon la revendication 1 ou 2, dans lequel ladite silicone est dans un domaine de concentration de 0,001 % (v/v) à 1 % (v/v).

4. Milieu de culture selon l'une quelconque des revendications 1 à 3, dans lequel ledit détergent est choisi parmi le groupe consistant en Tween 80™, Tween 20™, Triton WR-1339™, Triton X-100™, Triton X-405™, Triton X-114™, Triton N-101™, Triton VG-110™ et Triton XL-80N™.

5. Milieu de culture selon l'une quelconque des revendications 1 à 4, dans lequel ledit détergent est à la concentration de 0,01 % (v/v) à 1 % (v/v).

6. Milieu de culture selon la revendication 5, dans lequel ledit détergent est à la concentration de 0,3 % (v/v).

7. Milieu de culture selon l'une quelconque des revendications 1 à 6, lequel milieu comprend un milieu synthétique ou un milieu complexé.

8. Milieu de culture selon la revendication 7, dans lequel ledit milieu synthétique est un milieu Sauton, un milieu Kirchner ou un milieu Proskauer-Beck.

9. Milieu de culture selon la revendication 7, où ledit milieu complexé est un milieu Dubos, un milieu Ungar, un milieu Middlebrook 7H9, un milieu Middlebrook 7H11, un milieu Lowenstein/Lowenstein-Jensen ou un bouillon de sels de glycine-alanine (GAS).

10. Milieu de culture selon l'une quelconque des revendications 1 à 9, dans lequel lesdites mycobactéries sont choisies parmi le groupe consistant en M. tuberculosis, M. bovis, M. avium, M. africanum, M. kanasasii, M. intracellular, M. ulcerans, M. paratuberculosis, M. simiae, M. scrofulaceum, M. szulgai, M. xenopi, M. fortuitum, M. chelonei, M. leprae et M. marinum.

11. Milieu de culture selon la revendication 10, dans lequel ladite M. tuberculosis est la M. tuberculosis H37Rv, M. tuberculosis Erdman ou M. tuberculosis CSU93 et ladite M. bovis est la M. bovis BCG.

12. Utilisation d'un milieu de culture selon l'une quelconque des revendications 1 à 11 pour la culture de souches mycobactériennes destinées à la vaccination.

13. Utilisation du milieu de culture selon l'une quelconque des revendications 1 à 11 pour la culture de souches mycobactériennes destinées à une immunothérapie anticancéreuse ou une thérapie antiallergique.

14. Utilisation du milieu de culture selon l'une quelconque des revendications 1 à 11 pour la culture de souches mycobactériennes exprimant des protéines hétérologues.

15. Utilisation selon l'une quelconque des revendications 12 à 14, où ladite souche mycobactérienne est une souche atténuée.

16. Procédé destiné à la culture de mycobactéries dispersées consistant à faire croître lesdites mycobactéries dans le milieu de culture selon l'une quelconque des revendications 1 à 11.

17. Procédé selon la revendication 16, dans lequel le milieu de culture est agité durant la culture desdites mycobactéries.

18. Procédé selon la revendication 17, dans lequel ladite agitation est une agitation mécanique.

19. Procédé selon la revendication 17 ou 18, dans lequel ladite agitation comprend une agitation dans un domaine de 50 à 1000 tpm.

20. Procédé selon la revendication 19, dans lequel ladite agitation comprend une agitation à 360 à 500 tpm.

21. Procédé selon l'une quelconque des revendications 17 à 20, dans lequel la pression partielle d'oxygène (pO₂) est maintenue au-dessus de 5 % et en dessous de 80 % de saturation.

22. Procédé selon la revendication 21, dans lequel ladite pression partielle d'oxygène (pO₂) est maintenue entre 20 % et 35 % de saturation.

23. Procédé pour obtenir une cultures mycobactériennes standardisées comprenant les étapes consistant à
(a) mettre en culture une culture mycobactérienne potentiellement mixte dans le milieu selon l'une quelconque des revendications 1 à 11;
(b) isoler une cellule mycobactérienne dispersée et/ou unique; et
(c) faire croître ladite cellule mycobactérienne dispersée et/ou unique isolée dans un milieu frais.

24. Procédé pour la production d'un ou plusieurs dérivés protéiques purifiés (PPD) comprenant les étapes consistant à
(a) mettre en culture des mycobactéries dans le milieu de culture selon l'une quelconque des revendications 1 à 11 ; et
(b) isoler le PPD.

25. Utilisation de la culture mycobactérienne standardisée obtenue par le procédé selon la revendication 23 ou utilisation des mycobactéries dispersées mises en culture par le procédé selon l'une quelconque des revendications 16 à 22 pour la préparation d'une composition diagnostique pour tester l'efficacité d'un vaccin.

26. Utilisation de la culture mycobactérienne standardisée obtenue par le procédé selon la revendication 23 ou utilisation des mycobactéries dispersées mises en culture par le procédé selon l'une quelconque des revendications 16 à 22 pour la préparation d'une composition pharmaceutique pour la vaccination contre une maladie induite par des mycobactéries.

27. Utilisation de la culture mycobactérienne standardisée obtenue par le procédé selon la revendication 23 ou utilisation des mycobactéries dispersées mises en culture par le procédé selon l'une quelconque des revendications 16 à 22 pour la préparation d'une composition pharmaceutique destinée à une immunothérapie anticancéreuse ou une thérapie antiallergique.

28. Utilisation selon la revendication 26, où ladite maladie induite par des mycobactéries comprend la tuberculose, la lèpre ou des maladies de peau.

29. Kit comprenant le milieu de culture selon l'une quelconque des revendications 1 à 11.
